# EUROPEAN PATENT SPECIFICATION

(11) **EP 4 651 843 B1**
(45) Date of publication and mention of the grant of the patent: **18.03.2026**
(21) Application number: 23853583.5
(22) Date of filing: 19.12.2023
(51) Int. Cl.: A61F 5/058

(54) **ASSEMBLY OF A CRANIAL REMODELING ORTHOSIS**
ANORDNUNG EINER KRANIALEN REMODELLIERENDEN ORTHESE
ENSEMBLE ORTHÈSE DE REMODELAGE CRÂNIEN

(30) Priority: 18.01.2023 CZ 20230017
(43) Date of publication of application: 26.11.2025
(73) Proprietor: INVENT MEDICAL GROUP, S.R.O., 70800 Ostrava (CZ)
(72) Inventor: ROSICKY, Jan, 73911 Frydlant nad Ostravici, Frydlant (CZ); ROSICKY, Jiri, 73911 Frydlant nad Ostravici, Frydlant (CZ); MARTINEK, Matej, 74601 Opava (CZ)
(74) Representative: Tomickova, Dana
(86) International application number: PCT/CZ2023/050091
(87) International publication number: WO 2024/153273

(56) References cited:
- WO-A1-2017/042550
- WO-A1-2018/195602
- WO-A1-2018/212632
- CN-U- 217 448 164
- KR-B1- 102 355 799
- US-A1- 2021 322 200

## Description

### Technical Field

The invention relates to an assembly of a cranial remodeling orthosis, in particular for treatment of head deformations in newborns, infants and toddlers.

### Background of the Invention

Cranial remodeling orthoses are used to secure or support remodeling of the wearer's head into the proper shape. These orthoses must be precisely adapted to the head of the particular wearer for both their comfort and proper function of the cranial orthosis.

For example, newborns, infants, and toddlers sometimes experience unwanted head deformation (usually from lying often in one position) that requires prompt corrective treatment to correct the shape of the head. These deformations include the so-called plagiocephaly, which is a defect in the symmetry of the head, brachycephaly, which is a defect in the proportions of the head, or a combination of the two (referred to as asymmetrical brachycephaly), where the head exhibits both asymmetry and defective proportions. Plagiocephaly is manifested, for example, by asymmetrical flattening on the rear side of the head, prominence of the forehead on the side of the rear flattening, forward displacement of the ear on the side of the rear flattening, or asymmetry of the position of the face. Brachycephaly is manifested by a symmetrical flattening on the rear side of the head, an excessive width of the head, or a higher skull when viewed from above. The combination of these two types of deformation is then manifested by bilateral flattening of the rear side of the head, where one side of the head is flatter than the other, the forehead is displaced forward on the side of the greater flattening on the rear side of the skull, or the ear is displaced forward on the side of the greater occipital flattening.

These defects can be treated by wearing a cranial orthosis. Such an orthosis has an inner cavity to receive the head specifically designed to correspond to the shape of the head that the head should have at the end of treatment. Such an orthosis then directs the correction of the shape of the head during growth. This means that in some places the inner wall of the shell of the cranial orthosis is not in contact with the head, so head growth is supported in these places, while in places where the inner wall of the shell of the cranial orthosis is in contact with the head, head growth is limited. However, ensuring this ideal shape of the inner wall of the shell which ensures proper growth results in the fact that the cranial orthosis, by nature of its function, does not fit firmly on the wearer's head, wherein unwanted rotations of the orthosis relative to the head often occur, which results in less comfort for the wearer and less effectiveness of the cranial orthosis treatment.

Currently, there are several solutions to ensure a better fit of the cranial orthosis on the wearer's head. The problem of a better fit of the orthosis on the wearer's head is often solved by the fact that the shape of the inner wall of the shell is not adapted to the target shape that the head should have but only corresponds to a certain intermediate stage of growth towards the target shape. In such solutions, the shape of the inner cavity of the shell is adjusted such that the gap between the head and the inner wall of the shell is reduced in the places of deformations such that the orthosis does not allow such large unwanted rotational movements and at the same time growth is supported in the given place, i.e. the head is still not in contact with the inner wall of the shell in the place of deformation. The main disadvantage of this solution is the fact that the cranial orthosis has to be changed frequently, as after the head grows to the inner wall of the shell in the place of deformation, a new orthosis has to be created to again provide room for growth in the place of deformation. Thus, a large number of cranial orthoses must be used in the treatment process, corresponding to different intermediate stages in the growth to the target physiological shape of the head. The treatment thus requires a large amount of materials for the production of different shells, more specialist time is needed to plan the entire treatment, and the wearer often has to attend check-ups, making the treatment significantly more complicated and expensive.

These costs are reduced in solutions where the shell is made of a material that can be ground from the inside, wherein the specialist grinds the inside of the cranial orthosis in the course of the treatment such that the inner shape supports the growth in the places of deformation. Based on continuous check-ups, the inside of the orthosis is gradually ground to the ideal shape that the head should have. This process is technologically demanding and requires a high level of expertise, it is not possible to simply monitor and predict the course of the treatment, wherein it is always necessary to react to the actual shape of the wearer's head at the check-up. In addition, in the early stages of wear, the cranial orthosis is too heavy as it has to incorporate all the material that is gradually ground, which can lead to complications with the development of the neck and spine, possibly leading to their injury.

Another partial solution to the above-mentioned problems is a cranial orthosis with adjustable fastening, for example according to the document WO2019115965 A1. By means of the adjustable fastening of the helmet from the side, the helmet can be gradually opened as the head grows into the current volume of the inside of the shell. There is no need to modify the helmet in any way or to create new helmets all the time. However, such a solution can only be applied to some types of deformations and the effectiveness of such a solution is significantly limited. In addition, it is a structurally demanding solution and there is a risk of unwanted displacement of the clamping mechanism.

Other cranial orthoses are described in documents WO2017/042550 A1 and CN217448164 U. The cranial orthosis according to WO2017/042550 A1 comprises an inner mesh layer, which has a varying stiffness in different parts. The mesh layer is in a full contact and is stretching with a growing head during the whole period of wearing the orthosis. This document discloses an orthosis as defined in the preamble of claims 1 and 4. CN217448164 U describes a cranial orthosis with an outer shel and an inner orthopedic sheet, wherein the outer shell is formed from a single part.

For the above reasons, it is desirable to develop a cranial remodeling orthosis that sits firmly and comfortably on the wearer's head, providing correct treatment for a wide range of different head deformations during the whole period of wearing the cranial remodeling orthosis.

### Summary of the Invention

The above shortcomings are eliminated by an assembly of a cranial remodeling orthosis comprising a first front piece of a shell and a first rear piece of a shell adapted for mutual removable connection, wherein an inner wall of the first front piece of the shell and an inner wall of the first rear piece of the shell together define a target inner cavity of the cranial orthosis, wherein it further comprises a second rear piece of the shell adapted for removable connection to the first front piece of the shell, wherein the inner wall of the second rear piece of the shell and the inner wall of the first front piece together define a reduced inner cavity of the cranial orthosis that has a smaller volume than the target inner cavity of the cranial orthosis, wherein the second rear piece of the shell is replaced by the first rear piece of the shell to form the target inner cavity. This embodiment of the assembly of the invention is adapted to correct deformations on the rear side of the wearer's head.

The above shortcomings are eliminated by an assembly of a cranial remodeling orthosis comprising a first front piece of a shell and a first rear piece of the shell adapted for mutual removable connection, wherein an inner wall of the first front piece of the shell and an inner wall of the first rear piece of the shell together define a target inner cavity of the cranial orthosis, it further comprises a second front piece of the shell adapted for removable connection to the first rear piece of the shell, wherein the inner wall of the second front piece of the shell and the inner wall of the first rear piece of the shell together define a reduced inner cavity of the cranial orthosis that has a smaller volume than the target inner cavity of the cranial orthosis, wherein the second front piece of the shell is replaced by the first front piece of the shell to form the target inner cavity. This embodiment of the assembly of the invention is adapted to correct deformations on the front side of the wearer's head.

The advantage of the present invention of the cranial remodeling orthosis is that it sits firmly and comfortably on the wearer's head while providing proper treatment for a wide range of different head deformations, throughout the whole period of wearing such a cranial remodeling orthosis. The main idea is to create an inner cavity of the cranial orthosis corresponding to the intermediate stage of the growth of the head during the correction to the target, physiological shape of the wearer's head. Thus, in the first phase of the treatment, the cranial orthosis of the invention has a smaller inner cavity of the cranial orthosis, wherein it fits more stably on the wearer's head, and thus provides a higher-quality and more comfortable treatment with the remodeling orthosis. Two different inner cavities of the cranial orthosis are achieved by at least two front pieces or at least two rear pieces, depending on which regions of the head show deformations and where it is therefore preferable to reduce the inner cavity of the cranial orthosis during the remodeling.

Thus, the assembly of the cranial orthosis of the invention is composed of at least three pieces, the first front piece of the shell, the first rear piece of the shell forming the target inner wall of the cranial orthosis, and at least one second rear or front piece of the shell that, in combination with the respective first piece of the shell, forms the reduced inner cavity of the cranial orthosis. The shell refers to the basic load-bearing part of the cranial orthosis, which defines the outer shape of the orthosis and ensures sufficient firmness and structural stability of the orthosis. The pieces of the shell further comprise an inner wall of the piece of the shell that defines the inner cavity of the cranial orthosis that is adapted to contain the wearer's head. The front piece of the shell comprises the inner wall of the front piece of the shell that is adapted to the shape of at least a part of the front side of the wearer's head, the rear piece of the shell comprises the inner wall of the rear piece that is adapted to the shape of at least a part of the rear side of the wearer's head. Preferably, the basic structure of the pieces of the shell comprises an inner and outer envelope with a mesh 3D structure between them to reinforce the structure, where the inner envelope defines the inner wall of the cranial orthosis, and the outer envelope provides the outer shape of the orthosis and sufficient firmness and structural stability of the orthosis. In various embodiments, the front or rear piece of the shell may the composed of multiple pieces or may have various complimentary layers placed on the inner envelope, i.e. a lining or other layers providing, for example, a greater comfort or safety. These additional layers are placed on the inner envelope of the basic structure of the piece of the shell, thereby naturally affecting the shape of the inner wall of the cranial orthosis and therefore the shape of the inner cavity of the cranial orthosis for containing the head, wherein these additional layers must be considered in the design of the basic structure of the pieces of the shell. The inner wall of the front or rear piece means the wall defining the inner cavity of the cranial orthosis, which may be formed by the wall of the lining or directly the inner wall of the inner envelope of the basic structure of the piece of the shell. The basic structure of the piece of the shell means the load-bearing structure of the given piece, which forms the outer wall of the piece of the shell and the basis for the inner wall of the given piece, preferably the basic structure is of a rigid 3D printed material.

The inner cavity of the cranial orthosis for the insertion of the wearer's head means the space within the cranial orthosis that the cranial orthosis surrounds in shape and that is adapted to contain the wearer's head, this space thus roughly corresponds to the outer shape of the wearer's head. The inner cavity of the cranial orthosis is defined by the shell and its orifice corresponds to the marginal contours of the shell that in cranial remodeling orthoses run above the eyebrows, then on both sides of the head around the ears and meet in the region of the occipital bone. With its inner shape, the cranial remodeling orthosis, or rather the inner cavity of the cranial orthosis, roughly corresponds to the shape of the skull in the regions of the frontal bone, parietal bones, and occipital bone. From the inner side, the pieces of the shell may comprise a lining or other layers forming at least partially the inner wall of the pieces for a greater comfort or breathability of the cranial orthosis.

The front piece may be connected to the rear piece of the shell removably in various ways. Preferably, the first rear piece of the shell comprises a clamping mechanism for providing the removable connection to both the first front piece of the shell and the second front piece of the shell. Preferably, the first front piece of the shell comprises a clamping mechanism for providing a removable connection to both the first rear piece of the shell and the second rear piece of the shell. The front piece may be connected to the rear piece of the shell removably in various ways. Preferably, the front and rear pieces comprise a clamping mechanism providing the connection of the pieces of the shell on both the left and right side of the wearer's head. For example, the first front piece may comprise a locking element in form of a flat lever rotationally connected at one end thereof to the outer wall of the first front piece, with a tab attached to the lever. The first rear piece and the second rear piece then comprise a groove, or multiple grooves, for attaching a cable, wherein after inserting the cable into the groove and turning the flat lever towards the outer envelope of the first piece, the pieces are connected firmly together. The disassembly is then achieved by turning the flat lever to a position perpendicular to the outer wall of the first piece and removing the tab from the groove of the rear piece of the shell. Therefore, preferably the second rear piece of the shell comprises the same part of the clamping mechanism as the first rear piece and likewise in the case of the second front piece of the shell. In addition, the front and rear pieces may be connected by gluing, double-sided adhesive tape, or by a mechanical connection such as a groove-projection type, or via an elastic stop. The removable connection of the front and rear pieces of the shell generally means such an implementation of their connection that allows their separation without significant damage that would limit the functionality of the cranial orthosis.

The description of the inner wall of the cranial orthosis and the inner cavity of the cranial orthosis refers to the shell in the assembled position, where the respective front and rear pieces our put together.

In the context of the invention, the cranial orthosis has a variable inner cavity of the cranial orthosis, wherein the size of the inner cavity of the cranial orthosis may change when replacing the second piece of the shell (front or rear) with the corresponding first piece of the shell. In this context, two inner cavities of the cranial orthosis are defined: target and reduced. The reduced cavity of the cranial orthosis is defined by the reduced inner wall of the cranial orthosis and the target cavity of the cranial orthosis is defined by the target inner wall of the cranial orthosis, wherein the reduced cavity of the cranial orthosis has a smaller volume than the target cavity of the cranial orthosis, or the target cavity is adapted to contain a wearer's head with a larger volume. Thus, in general, the target and reduced cavities of the cranial orthosis correspond to different stages of head growth during the remodeling, where the reduced cavity corresponds to the growth intermediate stage in the growth of the head to the target shape of the wearer's head, which corresponds to the target inner cavity of the cranial orthosis. The reduced wall of the cranial orthosis may be formed together by the first front piece and the second rear piece or the second front piece and the first rear piece, or by their inner walls, and the target wall of the cranial orthosis is formed by the first front piece and the first rear piece, or by their inner walls. Additional layers such as the lining affect the shape of the inner wall of the piece of the shell, or the inner cavity of the cranial orthosis, wherein the inner wall of the inner envelope of the basic structure of the piece of the shell must be adapted with its shape to the given additional layers such that together they form the required shape of the inner wall of the piece of the shell, and hence the inner wall of the cranial orthosis.

The inner wall of the cranial orthosis generally differs from the initial outer shape of the wearer's head in places where the initial shape of the wearer's head is significantly smaller than the normal, physiological shape of the head that is the target of the treatment with the cranial remodeling orthosis, wherein in this place growth is to be supported by the orthosis, whereas in some regions the inner wall of the cranial orthosis may completely merge with the initial model of the outer shape of the wearer's head, wherein in these places the growth of the head is limited by the orthosis. The reduced inner wall of the cranial orthosis then corresponds to the shape of the head at a certain intermediate stage between the current shape of the head and the target physiological shape of the head, and the target inner wall of the cranial orthosis corresponds to the target physiological shape of the head.

The reduced inner cavity of the cranial orthosis corresponds to the volume of the target inner cavity of the cranial orthosis reduced by the volume that is removed from the target volume of the inner cavity by the inner wall of the respective second piece of the shell, which is subsequently replaced by the first piece of the shell that forms the target inner cavity of the cranial orthosis with the original first piece. Thus, the reduced inner cavity of the cranial orthosis means the target inner cavity of the cranial orthosis reduced by a certain volume that corresponds to the difference between the inner wall of the second piece and the inner wall of the first piece that replaces the second piece to form the target inner cavity of the cranial orthosis.

The inner wall of the piece of the shell has a generally concave or convex shape adapted to the shape of the head and defining the inner cavity of the outer shell that also corresponds to reduced or target inner cavity of the cranial orthosis.

Preferably, the pieces of the shell comprise a perforated inner wall of the piece of the shell. These inner walls may be rigid or flexible, wherein increased flexibility may be achieved by perforations in the inner wall of the piece of the shell or by selection of the material forming the inner wall of the piece of the shell. A flexible, soft implementation of the inner wall of the piece of the shell may be achieved by comprising a lining, or the inner wall of the cranial orthosis may be formed by a mesh 3D structure extending to a certain depth of the piece of the shell below the surface of its inner wall, which, due to the specific structure of the mesh, or due to a specific material, achieves a higher flexibility of the inner wall in the given region of the surface.

The mesh 3D structure comprises shaped openings distributed in its volume, the specific implementation of which can be used to modify the flexibility or stiffness of the wall of the piece of the shell, or only a part of it. Depending on the specific shape, distance, and size, the openings modify the stiffness of the given region and give it a different directional expansion. The openings are meant to be volume cutouts of a specific shape in the volume of the mesh 3D structure. The stiffness of the mesh 3D structure is determined by the specific shape, distance, and size of the openings located in the given volume. The openings are distributed in the volume of the mesh 3D structure such that the stiff structure filling the volume between them has the structure of a three-dimensional (3D) mesh. In the case where the openings are smaller, have a shape that provides a greater stiffness to the surrounding structure, and/or are at a distance from each other, thicker structures of the material that fills the space between the openings are achieved, thereby ensuring a higher stiffness of the mesh 3D structure. By thicker 3D printed structures are meant structures with a larger cross-section in the place between the openings and with a higher volume representation in proportion to the volume representation of the openings. If the openings have a larger shape that provides a lower stiffness to the surrounding structure, and/or are located close together, weaker structures of the material that fills the space between the openings are achieved, thereby ensuring a lower stiffness of the mesh 3D structure. By weaker 3D printed structures are meant structures with a smaller cross-section in the place between the openings and with a smaller volume representation relative to the volume representation of the openings.

The target and reduced cavity of the cranial orthosis can also be understood in the context of the invention as cavities defined not only by the inner wall of the cranial remodeling orthosis but also by the outer shape of the wearer's head, specifically the initial shape of the wearer's head. In other words, in general, the inner cavity of the cranial orthosis can be understood as the cavity between the outer shape of the wearer's head and the inner wall of the cranial orthosis. Thus, the target inner cavity of the cranial orthosis may be understood as the cavity between the initial outer shape of the wearer's head and the target inner wall of the cranial orthosis, and the reduced inner cavity of the cranial orthosis may be understood as the cavity between the initial outer shape of the wearer's head and the reduced inner wall of the cranial orthosis. In this sense, the reduced inner cavity of the cranial orthosis may be 10 to 90% smaller, preferably 40 to 60% smaller, than the target inner cavity of the cranial orthosis.

For the invention to function properly, it is necessary that the reduced inner wall corresponds at least partially to the target inner wall of the cranial orthosis when they are centered. The difference in their shape is required, mainly in the place of deformation where the reduced inner wall of the cranial orthosis at least partially reduces the space between the wearer's head and the target inner wall of the cranial orthosis. The inner walls of the pieces of the shell must form the reduced inner wall such that it corresponds to the intermediate stage of growth of the head in the place of deformation, while in the other regions of the head it is undesirable to support growth, since they have a shape that already corresponds to the target outer shape of the head, wherein in these other regions (which are not part of the deformation) the inner wall of the cranial orthosis is unchanged in both the reduced and the target version.

Preferably, the assembly of the first front piece of the shell and the first rear piece of the shell has the same outer shape as the assembly of the first front piece of the shell and the second rear piece of the shell. Similarly, analogously, the second front piece has the same outer shape as the first front piece. Thus, the cranial orthosis has the same outer shape throughout the treatment, which is preferable mainly in cases where the cranial orthosis needs to be equipped with some accessory from the outer side, this accessory will thus fit the cranial orthosis for the entire treatment period.

Preferably, the pieces of the shell are made of a 3D printed material, or preferably they are made by a 3D printing technology such as, for example, SLA, SLS, FDM, MJF, DLP, 3DP, PJF, CLIP technology. The material for production of the pieces of the shell can be, for example, polymers PA, ABS, PLA, PE, PP, CPP, HPP, PC, PETG, photopolymers, elastomers (TPU, TPA, TPE, silicones), and other materials suitable not only for the above-mentioned 3D printing methods. Preferably, a thermoplastic elastomer such as thermoplastic polyamide (TPA), thermoplastic polyurethane (TPU), or a copolymer blend (TPE) is used to produce the flexible regions including the 3D mesh structure.

The above shortcomings are eliminated by a method of designing and producing the assembly of the cranial remodeling orthosis of the invention comprises the steps of:
a) obtaining a model of the outer shape of the wearer's head,
b) designing a model of the assembly of the cranial remodeling orthosis based on the model of the outer shape of the wearer's head comprising a step of determining the target outer shape of the wearer's head based on the model of the outer shape of the wearer's head, a step of determining the target inner wall of the cranial orthosis defining the target inner cavity of the cranial orthosis based on the target outer shape of the wearer's head, and a step of designing the first front piece of the shell and the first rear piece of the shell based on the target inner wall of the cranial orthosis,
c) producing the assembly of the cranial orthosis,
wherein the step of designing the model of the cranial orthosis b) further comprises a step of determining the reduced inner wall of the cranial orthosis defining the reduced inner cavity of the cranial orthosis comprising a step of comparing the model of the outer shape of the wearer's head with a representation of the target outer shape of the wearer's head, wherein the step of designing the model of the cranial orthosis b) further comprises a step of designing the second rear piece of the shell based on the step of determining the reduced inner wall of the cranial orthosis, wherein the reduced inner wall of the cranial orthosis is at least partially formed by the inner wall of the second rear piece of the shell, and the step of producing the assembly of the cranial orthosis c) comprises a step of producing the second rear piece of the shell.

The above shortcomings are eliminated by a method of designing and producing the assembly of the cranial remodeling orthosis of the invention comprises the steps of:
a) obtaining a model of the outer shape of the wearer's head,
b) designing a model of the assembly of the cranial remodeling orthosis based on the model of the outer shape of the wearer's head comprising a step of determining the target outer shape of the wearer's head based on the model of the outer shape of the wearer's head, a step of determining the target inner wall of the cranial orthosis defining the target inner cavity of the cranial orthosis based on the target outer shape of the wearer's head, and a step of designing the first front piece of the shell and the first rear piece of the shell based on the target inner wall of the cranial orthosis,
c) producing the assembly of the cranial orthosis,
wherein the step of designing the model of the cranial orthosis b) further comprises a step of determining the reduced inner wall of the cranial orthosis defining the reduced inner cavity of the cranial orthosis comprising a step of comparing the model of the outer shape of the wearer's head with a representation of the target outer shape of the wearer's head, wherein the step of designing the model of the cranial orthosis b) further comprises a step of designing the second front piece of the shell based on the step of determining the reduced inner wall of the cranial orthosis, wherein the reduced inner wall of the cranial orthosis is at least partially formed by the inner wall of the second front piece of the shell, and the step of producing the assembly of the cranial orthosis c) comprises a step of producing the second front piece of the shell.

The initial step of the method of designing and producing the cranial remodeling orthosis of the invention is the step of obtaining the model of the outer shape of the wearer's head, or a 3D model based on the actual anatomical shape of the wearer's head, wherein this model can be obtained in several ways ranging from various scanning methods to entering the measured data into a computer device that designs the 3D model of the head based on the data. The wearer means the person for whom the cranial remodeling orthosis is intended. The most common method of obtaining the 3D model based on the anatomical shape of the wearer's head is a direct 3D scan of the wearer's head. During the 3D scan, the data obtained from the 3D scan of the wearer's head is transformed into a polygonal grid of defining points with a density of at least 10 points per cm². Another method is to make a physical model of the wearer's head, for example from a cast of the wearer's head, and a subsequent 3D scan of the created physical model. In some cases, it is advisable to modify the physical model of the head before the 3D scan. Other methods of obtaining the model of the outer shape of the wearer's head are, for example, digitizing the inner area of an existing cranial orthosis or making a physical model of the wearer's head from an existing cranial orthosis and subsequently digitizing the outer area of the obtained physical model of the head, which can be modified before the digitization. Furthermore, the model of the outer shape of the wearer's head can be obtained by first measuring the wearer's head, for example, its circumference, width, length, diagonal dimensions of the head, and these data are entered into a computer device based on user input, which designs a 3D model based on these data. The obtained 3D model based on the anatomical shape of the wearer's head is subsequently uploaded from the scanning device to the computer device, where it is further processed within the method of designing according to the invention.

The step of designing the model of the cranial orthosis follows, which comprises the steps of determining the target outer shape of the wearer's head, determining the target inner wall of the cranial orthosis, the step of determining the reduced inner wall of the cranial orthosis, and the step of designing the second front piece of the shell or the second rear piece of the shell. The step of determining the target outer shape of the wearer's head is performed based on the input parameters that comprise the model of the outer shape of the wearer's head. The model of the outer shape of the wearer's head can be modified before further steps are taken to better adapt to the specific needs of the wearer. Thus, the model of the outer shape of the wearer's head may mean a physical representation, a modified physical representation, a digital representation, or a modified digital representation of the outer shape of the wearer's head. Other input parameters may represent age, gender, type, and severity of the deformity, the wearer's medical data, planned length of the cranial orthosis treatment, width of the head in various places, length of the head, circumference of the head, shape/circumference percentile of the growth of the head, diagonal dimensions of the head, or other parameters representing the initial state of the wearer's head. Thus, the input parameters define the initial state of the wearer, in particular, the data that define the current deformation and affect the process of the remodeling of the head are important.

The target outer shape of the head is designed based on these input parameters, wherein it is based on the shape of the head that the head should have, i.e. the physiological shape of the head, taking into account the planned period of wearing the cranial remodeling orthosis by the wearer. The initial shape of the wearer's head, or the model of the outer shape of the wearer's head, shows the regions and types of deformations that need to be corrected. The most common corrections are a defect in the symmetry of the head - plagiocephaly, a defect in the proportions of the head - brachycephaly, or a combination of a defect in symmetry and a defect in proportions - a so-called combined deformity. In the centered position of the 3D model of the initial outer shape of the wearer's head and the 3D model of the target outer shape of the wearer's head, in some regions the 3D model of the target outer shape of the wearer's head is located above the area of the 3D model of the initial shape of the wearer's head, wherein in these regions the growth of the head will be supported, in other regions the area of the 3D model of the target outer shape of the wearer's head merges with the area of the 3D model of the initial outer shape of the wearer's head, wherein in these regions the growth will be at least partially limited.

The step of determining the target inner wall of the cranial orthosis is based on the step of determining the target outer shape of the wearer's head, wherein the resulting shape of the target inner wall is determined to achieve the target outer shape of the wearer's head. In some embodiments, the shape of the target inner wall of the cranial orthosis may directly correspond to the target outer shape of the wearer's head. In other embodiments, their shapes may differ, for example, the target inner wall of the cranial orthosis may be smaller in some regions, or may represent a less bulky shape of the head, wherein in these "reduced" regions the target inner wall of the cranial orthosis comprises flexible regions that may provide a firmer and more stable fit of the orthosis on the head, while at the same time these regions support the growth of the head to a certain extent corresponding to the extent of their flexibility.

The step of designing the model of the cranial orthosis further comprises the step of designing the first front piece and the step of designing the first rear piece of the shell. They are designed to comprise a sufficient structural basis for the designed implementations of the inner walls of the cranial orthosis. In these steps, it is evaluated whether the first front piece or the first rear piece will comprise any additional layers, for example a lining placed on the inner wall of the inner envelope of their basis structure. These layers and additional elements in the inner cavity of the orthosis affect the shape of the inner cavity of the cranial orthosis for insertion of the wearer's head, whether target or reduced, and therefore affect its function as a remodeling tool. All this information must be processed to create a customized lining and pieces of the shell that together form the ideal inner wall of the cranial orthosis that will be both comfortable and will represent the ideal remodeling tool to achieve a physiological shape of the head. Thus, in embodiments with the additional layer, the inner wall of the inner envelope of the basis structure of the first pieces of the shell may correspond to the shape of the target inner wall of the cranial orthosis increased by a thickness corresponding to the given additional layer, such as a lining, such that together they form a shape corresponding to the target inner wall of the cranial orthosis. The target inner wall of the cranial orthosis is achieved by placing the additional layer on the inner wall of the inner envelope of the basis structure of the first pieces of the shell. In embodiments without additional layers, the shape of the inner walls of the inner envelope of the basis structure of the first pieces of the shell may directly correspond to the target outer shape of the wearer's head.

Next, the step of designing the model of the cranial orthosis comprises the step of determining the reduced inner wall of the cranial orthosis. The step of determining the reduced inner wall of the cranial orthosis occurs similarly to the step of determining the target inner wall of the cranial orthosis, except that it is not based on the target outer shape of the wearer's head but on the shape of a certain intermediate stage of growth between the initial outer shape of the wearer's head and the target outer shape of the wearer's head. Thus, it can be said that the reduced inner wall of the cranial orthosis is designed based on a reduced model of the outer shape of the wearer's head that corresponds to this intermediate stage of growth. Therefore, the step of determining the reduced inner wall of the cranial orthosis comprises the step of comparing the model of the outer shape of the wearer's head with a representation of the target outer shape of the wearer's head. The representation of the target outer shape of the wearer's head may mean, in various embodiments, the target outer shape of the wearer's head, the target inner wall of the cranial orthosis, and the target inner cavity of the cranial orthosis, in digital or physical form. In some embodiments, the cranial remodeling orthosis with the target inner wall of the cranial orthosis comprising the first front piece and the first rear piece of the shell may be formed in advance of the design and production of the respective second piece of the shell, wherein in these cases, the representation of the target outer shape of the wearer's head may already be this target inner wall of the cranial orthosis based on the inner walls of the pieces of the shell of this formed cranial orthosis. In such embodiments, for example, the appropriate reduced inner wall of the cranial orthosis, or appropriate respective second piece of shell, may be determined by a trial-and-error method using pre-produced second pieces of the shell, where the best fit is tested in the step of comparing. The step of determining the reduced inner wall of the cranial orthosis and the step of designing and producing the second front piece of the shell or the second rear piece of the shell can thus occur simultaneously, or they can completely merge into one step. Preferably, however, this step of comparing occurs in a digitized form where the models are centered and the step of determining the reduced inner wall of the cranial orthosis occurs based on the space between the area of the digital 3D model of the initial shape of the wearer's head and the digital 3D model representation of the target outer shape of the wearer's head.

In some embodiments, the shape of the reduced inner wall of the cranial orthosis may directly correspond to the reduced outer shape of the wearer's head. In other embodiments, their shapes may differ, for example, the reduced inner wall of the cranial orthosis may be smaller in some regions, or may represent a less bulky shape of the head, wherein in these "reduced" regions the reduced inner wall of the cranial orthosis comprises flexible regions that may provide a firmer and more stable fit of the orthosis on the head, while at the same time these regions support the growth of the head to a certain extent corresponding to the extent of their flexibility.

In the step of determining the reduced inner wall of the cranial orthosis, the shape of the reduced inner wall of the cranial orthosis may be designed by averaging the area of the model of the outer shape of the wearer's head with the area of the representation of the target outer shape of the wearer's head, and thus the 3D model of the reduced inner wall of the cranial orthosis is formed by points corresponding to the average of these coordinates of these two areas representing the initial and target shapes of the wearer's head. In other embodiments, the reduced inner wall may be designed such that it is not entirely halfway between these areas as in the case of the average but closer to the area forming the model of the initial outer shape of the wearer's head or to the area of the model of the target outer shape of the wearer's head, wherein it may preferably be located at 1/3 to 2/3 of the distance from the area of the initial model to the area of the target model of the wearer's head. More preferably, the shape of the reduced inner wall of the cranial orthosis is located 1/3 to 1/2 of the distance from the area of the initial model to the area of the target model of the wearer's head. In specific dimensions, the largest gap between the model of the outer shape of the wearer's head and the representation of the target shape of the wearer's head in the place of deformation often reaches a distance of 10 to 25 mm, wherein the reduced inner wall of the cranial orthosis may be at a distance of 3.33 mm to 5 mm in the corresponding region in the case of a 10 mm gap and 8.33 mm to 12.5 mm in the case of a 25 mm gap between the model of the outer shape of the wearer's head and the representation of the target shape of the wearer's head in the place of deformation. In the other regions, the distances between the model of the outer shape of the wearer's head and the representation of the target shape of the wearer's head decrease in the direction from the region of the greatest deformation and, according to the given ratio, the distances relative to the reduced inner wall of the cranial orthosis decrease accordingly.

The methods of designing the reduced inner wall of the cranial orthosis presented above in the preceding paragraph are adapted to support growth uniformly in all the regions of deformation where the symmetry of the head, and possibly also the proportions of the head, are corrected by growth into the target inner wall of the cranial orthosis. In other preferred embodiments, for combined defects in symmetry and proportion of the wearer's head, the reduced inner wall of the cranial orthosis may be designed such that it corresponds to the outer shape of the wearer's head with corrected symmetry, wherein the inner shell and the formed reduced inner cavity of the cranial orthosis first correct the symmetry of the head and then, after replacing the second piece of the shell with the corresponding first piece of the shell, the growth of the head into the target inner cavity of the cranial orthosis corrects the proportions of the head. In other embodiments, first the proportions of the head and then the symmetry of the head may be solved analogously.

The step of designing the second rear piece of the shell is based on the step of designing the first front piece of the shell and the step of determining the reduced inner wall of the cranial orthosis. The step of designing the second front piece of the shell is based on the step of designing the first rear piece of the shell and the step of determining the reduced inner wall of the cranial orthosis. As part of the design of the second piece of the shell, the designed implementation of the corresponding first piece of the shell with which the second piece of the shell is to form the reduced inner wall of the cranial orthosis is evaluated. The inner wall of the second piece of the shell is designed to follow the inner wall of the corresponding first piece such that together they form the reduced inner wall of the cranial orthosis. In the steps of designing the second piece of the shell, it is further evaluated whether any additional layers, such as a lining, will be implemented. These layers and additional elements in the inner cavity of the orthosis affect the shape of the inner cavity of the cranial orthosis for insertion of the wearer's head, whether target or reduced, and therefore affect its function as a remodeling tool. All this information must be processed to create a customized lining and shell that together form the ideal inner wall of the cranial orthosis that will be both comfortable and will represent the ideal remodeling tool to achieve a healthy shape of the head. Thus, in some embodiments, the inner envelope of the basic structure of the second piece of the shell, or the surface thereof, may correspond to the shape of the reduced inner wall of the cranial orthosis increased by a thickness corresponding to the given additional layer, such as a lining, such that together they form a shape corresponding to the reduced inner wall of the cranial orthosis. In other words, the surface of the inner envelope of the basic structure of the second piece of the shell does not have to correspond to the shape of the reduced inner wall of the cranial orthosis, wherein the resulting shape of the reduced inner wall of the cranial orthosis is achieved by placing an additional layer on the surface of the inner shell. In embodiments where the second piece of the shell does not comprise additional layers, the shape of the surface of the inner envelope of the basic structure of the piece of the shell may directly correspond to the reduced outer shape of the wearer's head.

Preferably, in the steps of designing the second piece of the shell, the inner wall thereof is designed such that the regions of the inner wall of the second piece corresponding to the regions of the reduced inner wall of the cranial orthosis, by which the reduced inner wall of the cranial orthosis differs from the target inner wall of the cranial orthosis, are located exclusively in the space between the target inner wall of the cranial orthosis and the model of the outer shape of the wearer's head.

Preferably, in the step of comparing, a digitized 3D model of the outer shape of the wearer's head and a digitized 3D model of the target inner wall of the cranial orthosis are compared. In this way, it is possible to easily compare these models and design the reduced inner wall of the cranial orthosis more accurately without major deviations.

Preferably, the method of designing and producing the cranial remodeling orthosis comprises the step of obtaining the input parameters, wherein the input parameter is the model of the outer shape of the wearer's head and at least one parameter from the following set: age, gender, type, and severity of the deformity, planned length of the cranial orthosis treatment, width of the head, length of the head, circumference of the head, circumference percentile of the growth of the head, and diagonal dimensions of the head, wherein in the step of designing the model of the cranial remodeling orthosis, the model of the cranial remodeling orthosis is designed based on the input parameters. Age determines, among other things, the size of the head, its physiological shape, and the rate of growth of the head. These data can be used to improve the accuracy of the models of the inner walls of the cranial orthosis, wherein the rate of growth of the head determines when the head will grow to both the reduced and target shape, allowing for precise timing of the remodeling process and follow-ups with the specialist. The planned length of the treatment, especially in combination with the rate of growth, forms the data on which the specific implementation of the inner walls of the cranial orthosis is based. The circumference percentile of the growth of the head is a datum determining the curve of growth of the head over time, which is again a datum that the specific implementation of the inner walls of the cranial orthosis can be adapted to. The type and severity of the deformity may also serve to improve the accuracy of the model of the cranial orthosis, which may thus be reflected in specific implementations of the inner walls of the cranial orthosis, specifically through the implementation of the lining, flexible regions, and/or distances between the inner walls of the cranial orthosis and the wearer's head in the regions of the deformation.

Preferably, the step of producing the front and rear pieces of the shell is carried out by a 3D printing method. The 3D printing method makes it possible to produce the pieces of the shell of the invention at minimal initial cost.

Preferably, in the step of determining the reduced inner wall of the cranial orthosis, the reduced inner wall of the cranial orthosis is determined such that the volume between the model of the outer shape of the wearer's head and the reduced inner wall of the cranial orthosis is 10 to 90% smaller than the volume between the model of the outer shape of the wearer's head and the target inner wall of the cranial orthosis. More preferably, the volume between the model of the outer shape of the wearer's head and the reduced inner wall of the cranial orthosis is 40 to 60% smaller than the volume between the model of the outer shape of the wearer's head and the target inner wall of the cranial orthosis.

### Description of Drawings

A summary of the invention is further clarified using exemplary embodiments thereof, which are described with reference to the accompanying drawings, in which:
- Fig. 1: shows a lateral view of the assembly of the cranial remodeling orthosis according to the first exemplary embodiment of the invention,
- Fig. 2: shows a lateral section of the assembly of the cranial remodeling orthosis according to the first exemplary embodiment of the invention,
- Fig. 3: shows an upper section of the assembly of the cranial remodeling orthosis according to the first exemplary embodiment of the invention,
- Fig. 4: shows a lateral section of the assembly of the cranial remodeling orthosis according to the second exemplary embodiment of the invention,
- Fig. 5: shows an upper section of the assembly of the cranial remodeling orthosis according to the second exemplary embodiment of the invention,
- Fig. 6: shows a lateral section of the assembly of the first front piece and the second rear piece according to the second exemplary embodiment of the invention,
- Fig. 7: shows a lateral section of the assembly of the first front piece and the first rear piece according to the second exemplary embodiment of the invention,
- Fig. 8: shows a lateral section of the assembly of the cranial remodeling orthosis according to the third exemplary embodiment of the invention,
- Fig. 9: shows a diagram of the centered (initial) model of the outer shape of the wearer's head, the model of the reduced outer shape of the wearer's head, and the model of the target outer shape of the wearer's head.

### Exemplary embodiments of the invention

The assembly of the cranial remodeling orthosis of the invention will be further clarified using exemplary embodiments with reference to the respective drawings.

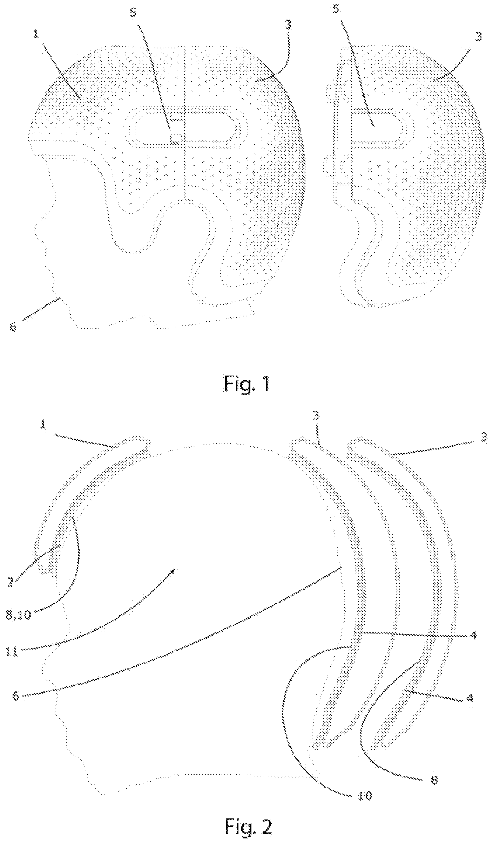

The first exemplary embodiment of the basic parts of the invention and its arrangement are illustrated in Fig. 1, Fig. 2, and Fig. 3. The assembly of the cranial orthosis of the first exemplary embodiment comprises a first front piece 1 of the shell, a first rear piece 3 of the shell, and a second rear piece 3 of the shell made of a 3D printed material, in particular polyamide. The inner wall 2 of the first front piece and the inner wall 4 of the first rear piece together form the target inner wall 8 of the cranial orthosis. The inner wall 2 of the first front piece and the inner wall 4 of the second rear piece together form the reduced inner wall 10 of the cranial orthosis. The first front piece 1 of the shell comprises a basic structure comprising an inner envelope and an outer envelope and a lining, where the lining is placed on the inner wall of the inner envelope of the basic structure of the first front piece 1 of the shell and forms the inner wall 2 of the first front piece of the shell. The first rear piece 2 of the shell comprises a basic structure comprising an inner envelope and an outer envelope and a lining, where the lining is placed on the inner wall of the inner envelope of the basic structure of the first rear piece 3 of the shell and forms the inner wall 4 of the first rear piece of the shell. The second rear piece 3 of the shell comprises a basic structure comprising an inner envelope and an outer envelope and a lining, where the lining is placed on the inner wall of the inner envelope of the basic structure of the secondary rear piece 3 of the shell and forms the inner wall 4 of the second rear piece of the shell. When designing the basic structure of the pieces of the shell, the thickness of the lining was considered such that after the insertion of the lining, together they would form the resulting inner wall of the piece of the shell, and therefore the given reduced or target inner wall 10,8 of the cranial orthosis.

In Fig. 1, the assembly of the first front piece 1 and the second rear piece 3 (left) and the separate first rear piece 3 of the shell (right) are illustrated. The first front piece 1 of the shell comprises, on the right and left sides, a first part of a clamping mechanism 5 of the cranial orthosis of the first exemplary embodiment, and the first and second rear pieces 3 of the shell comprise, on their left and right sides, a second part of the clamping mechanism 5 of the same implementation adapted for the connection to the first part of the clamping mechanism 5, thereby ensuring the connection of the pieces of the shell on the left and right sides of the wearer's head. The first part of the clamping mechanism 5 comprises a locking element in the form of a flat lever rotationally connected at one end thereof to the outer wall of the outer envelope of the shell of the first front piece 1, where a tab is attached to the lever. The second part of the clamping mechanism 5 then comprises a groove, or multiple grooves, for attaching a cable, wherein after inserting the cable of the first part of the clamping mechanism 5 into the groove and turning the flat lever towards the outer envelope of the front piece 1 of the shell, the front and rear pieces 1,3 of the shell are firmly connected together. The disassembly is then achieved by turning the flat lever to a position approximately perpendicular to the outer wall of the front piece 1 and removing the tab from the groove of the rear piece 3 of the shell. The second rear piece 3 of the shell comprises the same part of the clamping mechanism 5 as the first rear piece 3 of the shell.

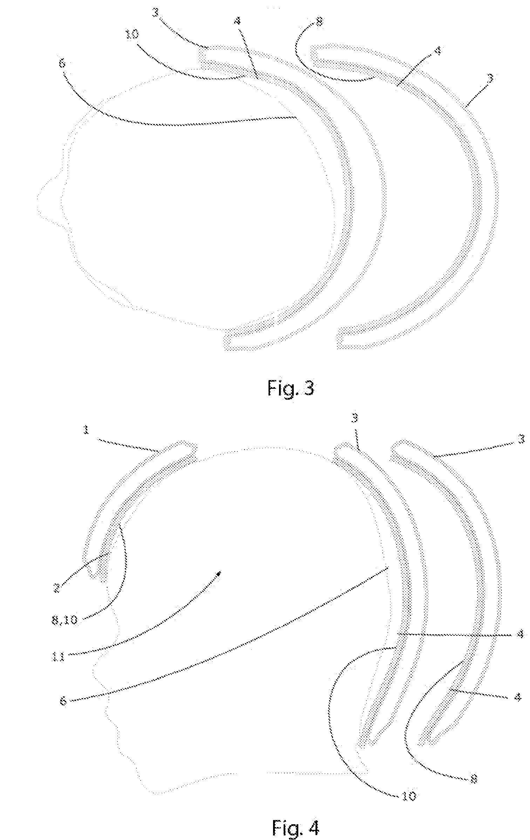

On a model 6 of the outer shape of the head illustrated in Fig. 2 and Fig. 3, it can be seen that the head comprises a significant deformation in the right rear part that is reduced towards the left rear side of the head, wherein on the left rear side of the head it has nearly a physiological, target shape. As can be seen in Fig. 2, the reduced inner cavity 11 is designed based on the outer shape of the wearer's head with corrected symmetry. The difference between the reduced inner wall 10 and the target inner wall 8 of the cranial orthosis is only in the length of the head, where the target inner wall 8 is slightly more recessed, their left and right sides are symmetrical, as can be seen in the upper section of Fig. 3. After achieving the shape of the wearer's head close to the shape of the reduced inner wall 10 of the cranial orthosis, a nearly corrected symmetry of the head is therefore expected. Therefore, the target inner wall 8 is then adapted to correct the proportions of the head.

The first rear piece 3 of the shell and the second rear piece 3 of the shell of the first exemplary embodiment comprise an identically shaped outer envelope of the basic structure, or they have the same outer shape, differing only in the shape of the inner envelope, thus also in the shape of the inner wall 4 of the rear piece of the shell, wherein the inner wall 4 of the first rear piece partially forms the target inner wall 8 of the cranial orthosis and the inner wall 4 of the second rear piece partially forms the reduced inner wall 10 of the cranial orthosis.

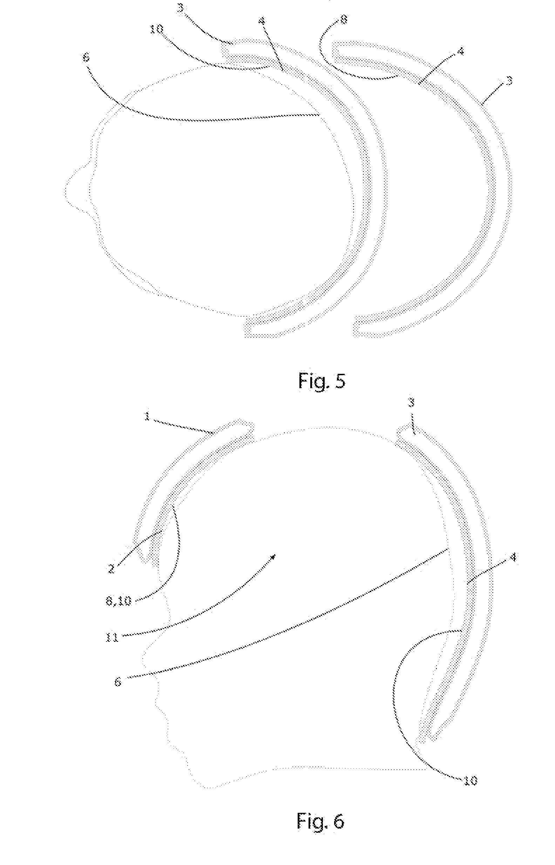

The assembly of the cranial orthosis of the second exemplary embodiment is illustrated in Fig. 4 to Fig. 7. This embodiment differs from the first exemplary embodiment in that the outer envelopes of the rear pieces 3 of the shell are adapted to the shape of the inner envelope, or their inner wall, such that the total thickness of the wall of the first rear piece 3 and the second rear piece 3 is approximately the same. This achieves that the assembly of the first front piece 1 and the second rear piece 3 of the shell has a smaller volume, and the cranial orthosis therefore does not take up as much space in the first part of the treatment with the reduced inner wall 10 of the cranial orthosis.

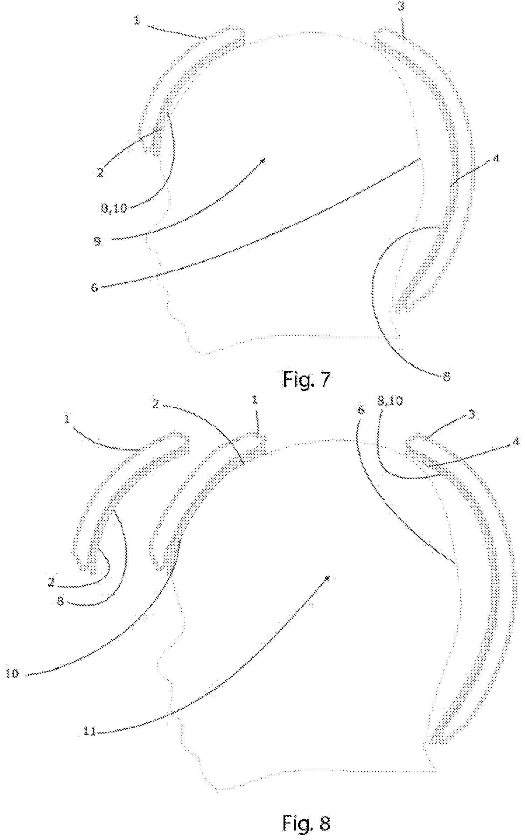

The assembly of the cranial orthosis of the third exemplary embodiment is illustrated in Fig. 8. This embodiment differs from the first exemplary embodiment in that instead of the second rear piece 3 of the shell, it comprises the second front piece 1 of the shell, where the inner wall 2 of the second front piece of the shell forms, together with the first rear piece 3 of the shell, the reduced inner wall 10 of the cranial orthosis. The model 6 of the outer shape of the wearer's head also shows signs of deformation in the front part of the head, and thus it is preferable to form the reduced inner wall 10 of the cranial orthosis differing from the target inner wall 8 of the cranial orthosis in the front part of the head, thereby reducing the gap between the wearer's head and the inner wall of the cranial remodeling orthosis, specifically by the second front piece 1, in the first part of the treatment, thereby minimizing unwanted rotations of the helmet.

All the drawings of the exemplary embodiments of the invention (Fig. 1 to Fig. 8) also show the initial model 6 of the outer shape of the wearer's head, wherein it can be observed how the first and second rear pieces 3 of the shell or the first and second front piece 1 of the shell change the size of the space between the initial outer shape of the wearer's head and the given inner wall of the cranial orthosis.

The method of designing and producing the cranial remodeling orthosis of the invention will be described herein using particular embodiments.

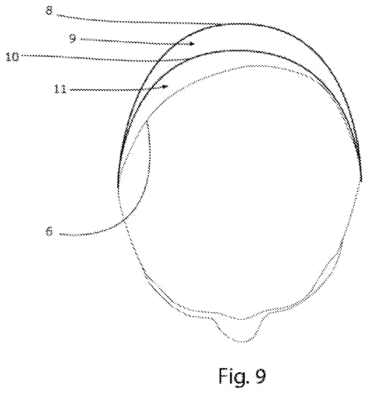

In an exemplary embodiment of the method of designing and producing the cranial remodeling orthosis of the invention, in the step of obtaining the model 6 of the outer shape of the wearer's head, the wearer's head is scanned by a scanning device, wherein the created 3D scan is stored in a memory of a computer device. This 3D scan of the wearer's head represents the initial model 6 of the outer shape of the wearer's head. The description of the relationship between the individual digitized areas (models 6 of the outer shape of the wearer's head, areas corresponding to the inner walls of the cranial orthosis, ...) is related to their centered position relative to the initial model 6 of the outer shape of the wearer's head, a schematic example of the centering of these areas is illustrated in Fig. 9.

The input parameters further comprise information that the target inner wall 8 of the cranial orthosis is to be provided with a 3 mm lining and information that correction of the symmetry of the head is preferred, followed by correction of the proportions of the head. Based on this model 6 of the outer shape of the wearer's head, the target outer shape 7 of the wearer's head is further designed, which is then modified to the final implementation of the target inner wall 8 of the cranial orthosis. Now, the shape of the target inner wall 8 of the cranial orthosis is known, and the design of the first front piece 1 and the first rear piece 3 of the shell on the computer device follows such that the assembly of the first pieces of the cranial orthosis in the result has the target shape of the inner wall of the cranial orthosis. A lining with a thickness of 3 mm will be placed on the inner wall of the inner envelope of the basic structure of the shell of the first pieces, therefore, the inner wall of the inner envelope of the basic structure of the shell of the first parts, which together with the lining is to define the target inner cavity 9 of the cranial orthosis, is increased (enlarged) by 3 mm over the area defining the target inner cavity 9 of the cranial orthosis, wherein the difference between the area defining the target inner cavity 9 of the cranial orthosis and the area defining the inner wall of the inner envelope of the basic structure of the shell of the first pieces corresponds to the dimensions of the lining. After the design of the parts of the pieces of the shell forming the inner walls 2,4 of the first rear piece and the first front piece of the shell, the rest of the structure of the first front piece 1 and the first rear piece 3, including the outer envelope of the basic structure of the front and rear pieces 1,3 of the shell that is structurally designed from the determined shape of the inner wall 2,4 of the front and rear pieces of the shell so as to give sufficient firmness and stability to the structure of the cranial orthosis, is designed.

The designed assembly of the first front piece 1 and the first rear piece 3 defining the target inner cavity 9 of the cranial orthosis is illustrated in Fig. 7, where a significant gap can be seen between the initial model 6 of the outer shape of the wearer's head and the target inner wall 8 of the cranial orthosis, resulting in unwanted rotations of the cranial orthosis around the wearer's head. Therefore, subsequently the second rear piece 3 of the shell is designed whose inner wall reduces this volume, and it is based on the reduced outer shape of the wearer's head, or the reduced inner wall 10 of the cranial orthosis.

The step of determining the reduced inner wall 10 of the cranial orthosis follows, which is schematically illustrated in Fig. 9. The step of determining the reduced inner wall 10 of the cranial orthosis comprises the step of comparing the model 6 of the outer shape of the wearer's head with the representation of the target outer shape 7 of the wearer's head, where the initial outer shape of the wearer's head is first compared with the target outer shape 7 of the wearer's head. In Fig. 9, it can be seen that the wearer's head has a significant deformation in the right rear region of the head, this deformation disturbs the overall symmetry of the head and its proportions. The reduced inner wall 10 of the cranial orthosis is to correspond to the shape of the head with corrected symmetry, wherein subsequently the defect in proportion will be addressed by growing the head into the target inner cavity 9 of the cranial orthosis. In this embodiment, the reduced inner wall 10 of the cranial orthosis is formed by modifying the target inner wall 8 of the cranial orthosis, which is modified by displacing it toward the model 6 of the initial outer shape of the wearer's head while maintaining a symmetrical shape. The reduced inner wall 10 of the cranial orthosis is designed here such that it divides the volume between the area corresponding to the initial outer shape of the wearer's head and the area corresponding to the target outer shape 7 of the wearer's head into two approximately equal cavities (see Fig. 9).

The step of designing the second rear piece 3 of the shell based on the reduced inner wall 10 of the cranial orthosis and based on the designed model of the first front piece 1 follows. A lining with a thickness of 3 mm will be placed on the inner wall of the inner envelope of the basic structure of second rear piece 3 of the shell, therefore, the inner wall of the inner envelope of the basic structure of the shell of the second rear piece 3, which together with the lining is to define the reduced inner cavity 11 of the cranial orthosis, is increased (enlarged) by 3 mm over the area defining the reduced inner cavity 11 of the cranial orthosis, wherein the difference between the area defining the reduced inner cavity 11 of the cranial orthosis and the area defining the inner wall of the inner envelope of the basic structure of the second piece of the shell corresponds to the dimensions of the lining. The first front piece 1 comprises a lining of the same thickness, wherein it is necessary to ensure that the inner walls of the inner envelopes of the first front piece 1 and the second rear piece 3 follow each other such that the transition between the inner wall 2 of the first front piece and the inner wall 4 of the second rear piece is perfectly smooth. After designing the parts of the second rear piece 3 of the shell forming the inner wall 4 of the second rear piece of the shell, the rest of the structure of the second rear piece 3, including the outer envelope of the basic structure of the second rear piece 3 of the shell that is structurally designed from the determined shape of the inner wall 4 of the second rear piece of the shell so as to give sufficient firmness and stability to the structure of the cranial orthosis, is designed.

Further, the clamping mechanism 5 is implemented on the outer envelopes of the basic structure of the pieces of the shell, following the first exemplary embodiment of the invention.

Subsequently, the designed first front piece 1 of the shell, the first rear piece 3 of the shell, and the second rear piece 3 of the shell are made by the 3D printing method from polyamide.

The resulting product of the exemplary method of designing and producing according to the invention is the assembly of the cranial orthosis according to the second exemplary embodiment illustrated in Fig. 4 to 7.

In an alternative embodiment, the assembly of the first front piece 1 and the second rear piece 3 of the shell may be first designed based on the step of determining the reduced inner wall 10 of the cranial orthosis, and subsequently a suitable first rear piece 3 of the shell may be designed in addition to the designed first front piece 1.

### Industrial Applicability

The invention can be used in the field of remodeling orthoses.

### List of Reference Signs

- 1 -: Front piece of the shell
- 2 -: Inner wall of the front piece of the shell
- 3 -: Rear piece of the shell
- 4 -: Inner wall of the rear piece of the shell
- 5 -: Clamping mechanism
- 6 -: Model of the outer shape of the wearer's head
- 7 -: Target outer shape of the wearer's head
- 8 -: Target inner wall of the cranial orthosis
- 9 -: Target inner cavity of the cranial orthosis
- 10 -: Reduced inner wall of the cranial orthosis
- 11 -: Reduced inner cavity of the cranial orthosis

## Claims

1. An assembly of a cranial remodeling orthosis comprising a first front piece (1) of a shell and a first rear piece (3) of the shell adapted for mutual removable connection, wherein an inner wall (2) of the first front piece of the shell and an inner wall (4) of the first rear piece of the shell together define a target inner cavity (9) of the cranial orthosis, **characterized in that** it further comprises a second rear piece (3) of the shell adapted for removable connection to the first front piece (1) of the shell, wherein the inner wall (4) of the second rear piece of the shell and the inner wall (2) of the first front piece together define a reduced inner cavity (11) of the cranial orthosis that has a smaller volume than the target inner cavity (9) of the cranial orthosis, wherein the second rear piece (3) of the shell is replaced by the first rear piece (3) of the shell to form the target inner cavity (9).

2. The assembly of the cranial remodeling orthosis according to claim 1, **characterized in that** the first front piece (1) of the shell comprises a clamping mechanism (5) for providing a removable connection to both the first rear piece (3) of the shell and the second rear piece (3) of the shell.

3. The assembly of the cranial remodeling orthosis according to claims 1 or 2, **characterized in that** the assembly of the first front piece (1) of the shell and the first rear piece (3) of the shell has the same outer shape of the shell as the assembly of the first front piece (1) of the shell and the second rear piece (3) of the shell.

4. An assembly of a cranial remodeling orthosis comprising a first front piece (1) of a shell and a first rear piece (3) of the shell adapted for mutual removable connection, wherein an inner wall (2) of the first front piece of the shell and an inner wall (4) of the first rear piece of the shell together define a target inner cavity (9) of the cranial orthosis, **characterized in that** it further comprises a second front piece (1) of the shell adapted for removable connection to the first rear piece (3) of the shell, wherein the inner wall (2) of the second front piece of the shell and the inner wall (4) of the first rear piece together define a reduced inner cavity (11) of the cranial orthosis that has a smaller volume than the target inner cavity (9) of the cranial orthosis, wherein the second front piece (1) of the shell is replaced by the first front piece (1) of the shell to form the target inner cavity (9).

5. The assembly of the cranial remodeling orthosis according to claim 4, **characterized in that** the first rear piece (3) of the shell comprises a clamping mechanism (5) for providing a removable connection to both the first front piece (1) of the shell and the second front piece (1) of the shell.

6. The assembly of the cranial remodeling orthosis according to claims 4 or 5, **characterized in that** the assembly of the first front piece (1) of the shell and the first rear piece (3) of the shell has the same outer shape of the shell as the assembly of the second front piece (1) of the shell and the first rear piece (3) of the shell.

7. A method of designing and producing the assembly of the cranial remodeling orthosis according to the preceding claims 1 to 3, comprising the steps of:
a) obtaining a model (6) of the outer shape of the wearer's head,
b) designing a model of the assembly of the cranial remodeling orthosis based on the model (6) of the outer shape of the wearer's head comprising a step of determining the target outer shape (7) of the wearer's head based on the model (6) of the outer shape of the wearer's head, a step of determining the target inner wall (8) of the cranial orthosis defining the target inner cavity (9) of the cranial orthosis based on the target outer shape (7) of the wearer's head, and a step of designing the first front piece (1) of the shell and the first rear piece (3) of the shell based on the target inner wall (8) of the cranial orthosis,
c) producing the assembly of the cranial orthosis,
**characterized in that**
the step of designing the model of the cranial orthosis b) further comprises a step of determining the reduced inner wall (10) of the cranial orthosis defining the reduced inner cavity (11) of the cranial orthosis comprising a step of comparing the model (6) of the outer shape of the wearer's head with a representation of the target outer shape (7) of the wearer's head, wherein the step of designing the model of the cranial orthosis b) further comprises a step of designing the second rear piece (3) of the shell based on the step of determining the reduced inner wall (10) of the cranial orthosis, wherein the reduced inner wall (10) of the cranial orthosis is at least partially formed by the inner wall (4) of the second rear piece of the shell, and the step of producing the assembly of the cranial orthosis c) comprises a step of producing the second rear piece (3) of the shell.

8. A method of designing and producing the assembly of the cranial remodeling orthosis according to the preceding claims 4 to 6, comprising the steps of:
a) obtaining a model (6) of the outer shape of the wearer's head,
b) designing a model of the assembly of the cranial remodeling orthosis based on the model (6) of the outer shape of the wearer's head comprising a step of determining the target outer shape (7) of the wearer's head based on the model (6) of the outer shape of the wearer's head, a step of determining the target inner wall (8) of the cranial orthosis defining the target inner cavity (9) of the cranial orthosis based on the target outer shape (7) of the wearer's head, and a step of designing the first front piece (1) of the shell and the first rear piece (3) of the shell based on the target inner wall (8) of the cranial orthosis,
c) producing the assembly of the cranial orthosis,
**characterized in that**
the step of designing the model of the cranial orthosis b) further comprises a step of determining the reduced inner wall (10) of the cranial orthosis defining the reduced inner cavity (11) of the cranial orthosis comprising a step of comparing the model (6) of the outer shape of the wearer's head with a representation of the target outer shape (7) of the wearer's head, wherein the step of designing the model of the cranial orthosis b) further comprises a step of designing the second front piece (1) of the shell based on the step of determining the reduced inner wall (10) of the cranial orthosis, wherein the reduced inner wall (10) of the cranial orthosis is at least partially formed by the inner wall (2) of the second front piece of the shell, and the step of producing the assembly of the cranial orthosis c) comprises a step of producing the second front piece (1) of the shell.

9. The method of designing and producing the cranial remodeling orthosis according to claim 7 or 8, **characterized in that** in the step of comparing, a digitized 3D model (6) of the outer shape of the wearer's head and a digitized 3D model of the target inner wall (8) of the cranial orthosis are compared.

10. The method of designing and producing the cranial remodeling orthosis according to claims 7 to 9, **characterized in that** it comprises the step of obtaining the input parameters, wherein an input parameter is the model (6) of the outer shape of the wearer's head and at least one parameter from the following set: age, gender, type, and severity of the deformity, planned length of the cranial orthosis treatment, width of the head, length of the head, circumference of the head, circumference percentile of the growth of the head, diagonal dimensions of the head, wherein in the step of designing the model of the cranial remodeling orthosis, the model of the cranial remodeling orthosis is designed based on the input parameters.

11. The method of designing and producing the cranial remodeling orthosis according to claims 7 to 10, **characterized in that** the step of designing the model of the cranial orthosis b) comprises the step of modifying the model (6) of the outer shape of the wearer's head.

12. The method of designing and producing the cranial remodeling orthosis according to claims 7 to 11, **characterized in that** in the step of determining the reduced inner wall (10) of the cranial orthosis, the reduced inner wall (10) of the cranial orthosis is determined such that the volume between the model (6) of the outer shape of the wearer's head and the reduced inner wall (10) of the cranial orthosis is 10 to 90% smaller than the volume between the model (6) of the outer shape of the wearer's head and the target inner wall (8) of the cranial orthosis.

## Patentansprüche

1. Eine Anordnung einer kranialen remodellierenden Orthese, umfassend ein erstes Vorderteil (1) einer Schale und ein erstes Hinterteil (3) der Schale, die für eine gegenseitige lösbare Verbindung ausgelegt sind, wobei eine Innenwand (2) des ersten Vorderteils der Schale und eine Innenwand (4) des ersten Hinterteils der Schale zusammen einen Zielinnenhohlraum (9) der kranialen Orthese definieren, **dadurch gekennzeichnet, dass** sie ferner ein zweites Hinterteil (3) der Schale umfasst, das für eine lösbare Verbindung mit dem ersten Vorderteil (1) der Schale ausgelegt ist, wobei die Innenwand (4) des zweiten Hinterteils der Schale und die Innenwand (2) des ersten Vorderteils zusammen einen reduzierten Innenhohlraum (11) der kranialen Orthese definieren, der ein kleineres Volumen als der Zielinnenhohlraum (9) der kranialen Orthese aufweist, wobei das zweite Hinterteil (3) der Schale durch das erste Hinterteil (3) der Schale ersetzt wird, um den Zielinnenhohlraum (9) zu bilden.

2. Die Anordnung der kranialen remodellierenden Orthese nach Anspruch 1, **dadurch gekennzeichnet, dass** das erste Vorderteil (1) der Schale einen Klemmmechanismus (5) umfasst, um eine lösbare Verbindung sowohl mit dem ersten Hinterteil (3) der Schale als auch mit dem zweiten Hinterteil (3) der Schale bereitzustellen.

3. Die Anordnung der kranialen remodellierenden Orthese nach Anspruch 1 oder 2, **dadurch gekennzeichnet, dass** die Anordnung aus dem ersten Vorderteil (1) der Schale und dem ersten Hinterteil (3) der Schale die gleiche Außenform der Schale aufweist wie die Anordnung aus dem ersten Vorderteil (1) der Schale und dem zweiten Hinterteil (3) der Schale.

4. Eine Anordnung einer kranialen remodellierenden Orthese, umfassend ein erstes Vorderteil (1) einer Schale und ein erstes Hinterteil (3) der Schale, die für eine gegenseitige lösbare Verbindung ausgelegt sind, wobei eine Innenwand (2) des ersten Vorderteils der Schale und eine Innenwand (4) des ersten Hinterteils der Schale zusammen einen Zielinnenhohlraum (9) der kranialen Orthese definieren, **dadurch gekennzeichnet, dass** sie ferner ein zweites Vorderteil (1) der Schale umfasst, das für eine lösbare Verbindung mit dem ersten Hinterteil (3) der Schale ausgelegt ist, wobei die Innenwand (2) des zweiten Vorderteils der Schale und die Innenwand (4) des ersten Hinterteils zusammen einen reduzierten Innenhohlraum (11) der kranialen Orthese definieren, der ein kleineres Volumen als der Zielinnenhohlraum (9) der kranialen Orthese aufweist, wobei das zweite Vorderteil (1) der Schale durch das erste Vorderteil (1) der Schale ersetzt wird, um den Zielinnenhohlraum (9) zu bilden.

5. Die Anordnung der kranialen remodellierenden Orthese nach Anspruch 4, **dadurch gekennzeichnet, dass** das erste Hinterteil (3) der Schale einen Klemmmechanismus (5) umfasst, um eine lösbare Verbindung sowohl mit dem ersten Vorderteil (1) der Schale als auch mit dem zweiten Vorderteil (1) der Schale bereitzustellen.

6. Die Anordnung der kranialen remodellierenden Orthese nach den Ansprüchen 4 oder 5, **dadurch gekennzeichnet, dass** die Anordnung aus dem ersten Vorderteil (1) der Schale und dem ersten Hinterteil (3) der Schale die gleiche Außenform der Schale aufweist wie die Anordnung aus dem zweiten Vorderteil (1) der Schale und dem ersten Hinterteil (3) der Schale.

7. Ein Verfahren zum Entwerfen und Herstellen der Anordnung der kranialen remodellierenden Orthese nach den vorstehenden Ansprüchen 1 bis 3, umfassend die folgenden Schritte:
a) Erhalten eines Modells (6) der Außenform des Kopfes des Trägers,
b) Entwerfen eines Modells der Anordnung der kranialen remodellierenden Orthese auf der Grundlage des Modells (6) der Außenform des Kopfes des Trägers, umfassend einen Schritt des Bestimmens der Zielaußenform (7) des Kopfes des Trägers auf der Grundlage des Modells (6) der Außenform des Kopfes des Trägers, einen Schritt des Bestimmens der Zielinnenwand (8) der kranialen Orthese, die den Zielinnenhohlraum (9) der kranialen Orthese definiert, auf der Grundlage der Zielaußenform (7) des Kopfes des Trägers, und einen Schritt des Entwerfens des ersten Vorderteils (1) der Schale und des ersten Hinterteils (3) der Schale auf der Grundlage der Zielinnenwand (8) der kranialen Orthese,
c) Herstellen der Anordnung der kranialen Orthese,
**dadurch gekennzeichnet, dass**
der Schritt b) des Entwerfens des Modells der kranialen Orthese ferner einen Schritt des Bestimmens der reduzierten Innenwand (10) der kranialen Orthese umfasst, die den reduzierten Innenhohlraum (11) der kranialen Orthese definiert, umfassend einen Schritt des Vergleichens des Modells (6) der Außenform des Kopfes des Trägers mit einer Darstellung der Zielaußenform (7) des Kopfes des Trägers, wobei der Schritt b) des Entwerfens des Modells der kranialen Orthese ferner einen Schritt des Entwerfens des zweiten Hinterteils (3) der Schale auf der Grundlage des Schritts des Bestimmens der reduzierten Innenwand (10) der kranialen Orthese umfasst, wobei die reduzierte Innenwand (10) der kranialen Orthese zumindest teilweise durch die Innenwand (4) des zweiten Hinterteils der Schale gebildet wird, und der Schritt c) des Herstellens der Anordnung der kranialen Orthese einen Schritt des Herstellens des zweiten Hinterteils (3) der Schale umfasst.

8. Ein Verfahren zum Entwerfen und Herstellen der Anordnung der kranialen remodellierenden Orthese nach den vorstehenden Ansprüchen 4 bis 6, umfassend die folgenden Schritte:
a) Erhalten eines Modells (6) der Außenform des Kopfes des Trägers,
b) Entwerfen eines Modells der Anordnung der kranialen remodellierenden Orthese auf der Grundlage des Modells (6) der Außenform des Kopfes des Trägers, umfassend einen Schritt des Bestimmens der Zielaußenform (7) des Kopfes des Trägers auf der Grundlage des Modells (6) der Außenform des Kopfes des Trägers, einen Schritt des Bestimmens der Zielinnenwand (8) der kranialen Orthese, die den Zielinnenhohlraum (9) der kranialen Orthese definiert, auf der Grundlage der Zielaußenform (7) des Kopfes des Trägers, und einen Schritt des Entwerfens des ersten Vorderteils (1) der Schale und des ersten Hinterteils (3) der Schale auf der Grundlage der Zielinnenwand (8) der kranialen Orthese,
c) Herstellen der Anordnung der kranialen Orthese,
**dadurch gekennzeichnet, dass**
der Schritt b) des Entwerfens des Modells der kranialen Orthese ferner einen Schritt des Bestimmens der reduzierten Innenwand (10) der kranialen Orthese umfasst, die den reduzierten Innenhohlraum (11) der kranialen Orthese definiert, umfassend einen Schritt des Vergleichens des Modells (6) der Außenform des Kopfes des Trägers mit einer Darstellung der Zielaußenform (7) des Kopfes des Trägers, wobei der Schritt b) des Entwerfens des Modells der kranialen Orthese ferner einen Schritt des Entwerfens des zweiten Vorderteils (1) der Schale auf der Grundlage des Schritts des Bestimmens der reduzierten Innenwand (10) der kranialen Orthese umfasst, wobei die reduzierte Innenwand (10) der kranialen Orthese zumindest teilweise durch die Innenwand (2) des zweiten Vorderteils der Schale gebildet wird, und der Schritt c) des Herstellens der Anordnung der kranialen Orthese einen Schritt des Herstellens des zweiten Vorderteils (1) der Schale umfasst.

9. Das Verfahren zum Entwerfen und Herstellen der kranialen remodellierenden Orthese nach Anspruch 7 oder 8, **dadurch gekennzeichnet, dass** im Schritt des Vergleichens ein digitalisiertes 3D-Modell (6) der Außenform des Kopfes des Trägers und ein digitalisiertes 3D-Modell der Zielinnenwand (8) der kranialen Orthese verglichen werden.

10. Das Verfahren zum Entwerfen und Herstellen der kranialen remodellierenden Orthese nach den Ansprüchen 7 bis 9, **dadurch gekennzeichnet, dass** es den Schritt des Erhaltens der Eingabeparameter umfasst, wobei ein Eingabeparameter das Modell (6) der Außenform des Kopfes des Trägers und mindestens ein Parameter aus dem folgenden Satz ist: Alter, Geschlecht, Art und Schweregrad der Deformität, geplante Dauer der Behandlung mit der kranialen Orthese, Breite des Kopfes, Länge des Kopfes, Umfang des Kopfes, Perzentil des Kopfumfangswachstums, diagonale Abmessungen des Kopfes, wobei im Schritt des Entwerfens des Modells der kranialen remodellierenden Orthese das Modell der kranialen remodellierenden Orthese auf der Grundlage der Eingabeparameter entworfen wird.

11. Das Verfahren zum Entwerfen und Herstellen der kranialen remodellierenden Orthese nach den Ansprüchen 7 bis 10, **dadurch gekennzeichnet, dass** der Schritt b) des Entwerfens des Modells der kranialen Orthese den Schritt des Modifizierens des Modells (6) der Außenform des Kopfes des Trägers umfasst.

12. Das Verfahren zum Entwerfen und Herstellen der kranialen remodellierenden Orthese nach den Ansprüchen 7 bis 11, **dadurch gekennzeichnet, dass** in dem Schritt des Bestimmens der reduzierten Innenwand (10) der kranialen Orthese die reduzierte Innenwand (10) der kranialen Orthese so bestimmt wird, dass das Volumen zwischen dem Modell (6) der Außenform des Kopfes des Trägers und der reduzierten Innenwand (10) der kranialen Orthese 10 bis 90 % kleiner ist als das Volumen zwischen dem Modell (6) der Außenform des Kopfes des Trägers und der Zielinnenwand (8) der kranialen Orthese.

## Revendications

1. Un ensemble d'orthèse de remodelage crânien, comprenant une première partie avant (1) d'une coque et une première partie arrière (3) de la coque adaptées pour être reliées de manière amovible l'une à l'autre, où une paroi intérieure (2) de la première partie avant de la coque et une paroi intérieure (4) de la première partie arrière de la coque définissent ensemble une cavité intérieure cible (9) de l'orthèse crânienne, **caractérisé en ce qu'**il comprend en outre une deuxième partie arrière (3) de la coque adaptée pour être reliée de manière amovible à la première partie avant (1) de la coque, où la paroi intérieure (4) de la deuxième partie arrière de la coque et la paroi intérieure (2) de la première partie avant définissent ensemble une cavité intérieure réduite (11) de l'orthèse crânienne qui a un volume plus petit que la cavité intérieure cible (9) de l'orthèse crânienne, où la deuxième partie arrière (3) de la coque est remplacée par la première partie arrière (3) de la coque pour former la cavité intérieure cible (9).

2. L'ensemble d'orthèse de remodelage crânien selon la revendication 1, **caractérisé en ce que** la première partie avant (1) de la coque comprend un mécanisme de serrage (5) pour fournir une connexion amovible à la fois à la première partie arrière (3) de la coque et à la deuxième partie arrière (3) de la coque.

3. L'ensemble d'orthèse de remodelage crânien selon les revendications 1 ou 2, **caractérisé en ce que** l'ensemble de la première partie avant (1) de la coque et de la première partie arrière (3) de la coque présente la même forme extérieure de la coque que l'ensemble de la première partie avant (1) de la coque et de la deuxième partie arrière (3) de la coque.

4. Un ensemble d'orthèse de remodelage crânien, comprenant une première partie avant (1) d'une coque et une première partie arrière (3) de la coque adaptées pour être reliées de manière amovible l'une à l'autre, où une paroi intérieure (2) de la première partie avant de la coque et une paroi intérieure (4) de la première partie arrière de la coque définissent ensemble une cavité intérieure cible (9) de l'orthèse crânienne, **caractérisé en ce qu'**il comprend en outre une deuxième partie avant (1) de la coque adaptée pour être reliée de manière amovible à la première partie arrière (3) de la coque, où la paroi intérieure (2) de la deuxième partie avant de la coque et la paroi intérieure (4) de la première partie arrière définissent ensemble une cavité intérieure réduite (11) de l'orthèse crânienne qui a un volume plus petit que la cavité intérieure cible (9) de l'orthèse crânienne, où la deuxième partie avant (1) de la coque est remplacée par la première partie avant (1) de la coque pour former la cavité intérieure cible (9).

5. L'ensemble d'orthèse de remodelage crânien selon la revendication 4, **caractérisé en ce que** la première partie arrière (3) de la coque comprend un mécanisme de serrage (5) pour fournir une connexion amovible à la fois à la première partie avant (1) de la coque et à la deuxième partie avant (1) de la coque.

6. L'ensemble d'orthèse de remodelage crânien selon les revendications 4 ou 5, **caractérisé en ce que** l'ensemble de la première partie avant (1) de la coque et de la première partie arrière (3) de la coque présente la même forme extérieure de la coque que l'ensemble de la deuxième partie avant (1) de la coque et de la première partie arrière (3) de la coque.

7. Un procédé de conception et de fabrication de l'ensemble de l'orthèse de remodelage crânien selon les revendications 1 à 3 précédentes, comprenant les étapes suivantes consistant à :
a) obtenir un modèle (6) de la forme extérieure de la tête du porteur,
b) concevoir un modèle de l'ensemble de l'orthèse de remodelage crânien sur la base du modèle (6) de la forme extérieure de la tête du porteur, comprenant une étape consistant à déterminer la forme extérieure cible (7) de la tête du porteur sur la base du modèle (6) de la forme extérieure de la tête du porteur, une étape consistant à déterminer la paroi intérieure cible (8) de l'orthèse crânienne définissant la cavité intérieure cible (9) de l'orthèse crânienne sur la base de la forme extérieure cible (7) de la tête du porteur, et une étape consistant à concevoir la première partie avant (1) de la coque et la première partie arrière (3) de la coque sur la base de la paroi intérieure cible (8) de l'orthèse crânienne,
c) fabriquer l'ensemble de l'orthèse crânienne,
**caractérisé en ce que**
l'étape b) consistant à concevoir le modèle de l'orthèse crânienne comprend en outre une étape consistant à déterminer la paroi intérieure réduite (10) de l'orthèse crânienne définissant la cavité intérieure réduite (11) de l'orthèse crânienne, une étape consistant à comparer le modèle (6) de la forme extérieure de la tête du porteur avec une représentation de la forme extérieure cible (7) de la tête du porteur, où l'étape b) consistant à concevoir le modèle de l'orthèse crânienne comprend en outre une étape consistant à concevoir la deuxième partie arrière (3) de la coque sur la base de l'étape consistant à déterminer la paroi intérieure réduite (10) de l'orthèse crânienne, où la paroi intérieure réduite (10) de l'orthèse crânienne est au moins partiellement formée par la paroi intérieure (4) de la deuxième partie arrière de la coque, et où l'étape c) consistant à fabriquer l'ensemble de l'orthèse crânienne comprend une étape consistant à fabriquer la deuxième partie arrière (3) de la coque.

8. Un procédé de conception et de fabrication de l'ensemble de l'orthèse de remodelage crânien selon les revendications 4 à 6 précédentes, comprenant les étapes suivantes consistant à :
a) obtenir un modèle (6) de la forme extérieure de la tête du porteur,
b) concevoir un modèle de l'ensemble de l'orthèse de remodelage crânien sur la base du modèle (6) de la forme extérieure de la tête du porteur, comprenant une étape consistant à déterminer la forme extérieure cible (7) de la tête du porteur sur la base du modèle (6) de la forme extérieure de la tête du porteur, une étape consistant à déterminer la paroi intérieure cible (8) de l'orthèse crânienne définissant la cavité intérieure cible (9) de l'orthèse crânienne sur la base de la forme extérieure cible (7) de la tête du porteur, et une étape consistant à concevoir la première partie avant (1) de la coque et la première partie arrière (3) de la coque sur la base de la paroi intérieure cible (8) de l'orthèse crânienne,
c) fabriquer l'ensemble de l'orthèse crânienne,
**caractérisé en ce que**
l'étape b) consistant à concevoir le modèle de l'orthèse crânienne comprend en outre une étape consistant à déterminer la paroi intérieure réduite (10) de l'orthèse crânienne définissant la cavité intérieure réduite (11) de l'orthèse crânienne, une étape consistant à comparer le modèle (6) de la forme extérieure de la tête du porteur avec une représentation de la forme extérieure cible (7) de la tête du porteur, où l'étape b) consistant à concevoir le modèle de l'orthèse crânienne comprend en outre une étape consistant à concevoir la deuxième partie avant (1) de la coque sur la base de l'étape consistant à déterminer la paroi intérieure réduite (10) de l'orthèse crânienne, où la paroi intérieure réduite (10) de l'orthèse crânienne est au moins partiellement formée par la paroi intérieure (2) de la deuxième partie avant de la coque, et où l'étape c) consistant à fabriquer l'ensemble de l'orthèse crânienne comprend une étape consistant à fabriquer la deuxième partie avant (1) de la coque.

9. Le procédé de conception et de fabrication de l'orthèse de remodelage crânien selon la revendication 7 ou 8, **caractérisé en ce que,** dans l'étape consistant à comparer, un modèle 3D numérisé (6) de la forme extérieure de la tête du porteur et un modèle 3D numérisé de la paroi intérieure cible (8) de l'orthèse crânienne sont comparés.

10. Le procédé de conception et de fabrication de l'orthèse de remodelage crânien selon les revendications 7 à 9, **caractérisé en ce qu'**il comprend l'étape consistant à obtenir les paramètres d'entrée, où un paramètre d'entrée est le modèle (6) de la forme extérieure de la tête du porteur et au moins un paramètre parmi l'ensemble suivant : âge, sexe, type et gravité de la déformation, durée prévue du traitement par orthèse crânienne, largeur de la tête, longueur de la tête, circonférence de la tête, percentile de circonférence de la croissance de la tête, dimensions diagonales de la tête, où, lors de l'étape consistant à concevoir le modèle de l'orthèse de remodelage crânien, le modèle de l'orthèse de remodelage crânien est conçu sur la base des paramètres d'entrée.

11. Le procédé de conception et de fabrication de l'orthèse de remodelage crânien selon les revendications 7 à 10, **caractérisé en ce que** l'étape b) consistant à concevoir le modèle de l'orthèse crânienne comprend l'étape consistant à modifier le modèle (6) de la forme extérieure de la tête du porteur.

12. Le procédé de conception et de fabrication de l'orthèse de remodelage crânien selon les revendications 7 à 11, **caractérisé en ce que,** dans l'étape consistant à déterminer la paroi intérieure réduite (10) de l'orthèse crânienne, la paroi intérieure réduite (10) de l'orthèse crânienne est déterminée de telle sorte que le volume entre le modèle (6) de la forme extérieure de la tête du porteur et la paroi intérieure réduite (10) de l'orthèse crânienne soit de 10 à 90 % plus petit que le volume entre le modèle (6) de la forme extérieure de la tête du porteur et la paroi intérieure cible (8) de l'orthèse crânienne.
